# EUROPEAN PATENT SPECIFICATION

(11) **EP 0 992 583 B1**
(45) Date of publication and mention of the grant of the patent: **26.03.2003**
(21) Application number: 99115046.7
(22) Date of filing: 04.08.1999
(51) Int. Cl.: C12N 15/10

(54) **Novel method for purifying covalently closed circular DNA**
Methode für die Reinigung kovalent geschlossener zirkulärer DNS
Methode pour la purification d'ADN circulaire fermé

(30) Priority: 14.09.1998 EP 98117389
(43) Date of publication of application: 12.04.2000
(73) Proprietor: QIAGEN GmbH, 40724 Hilden (DE)
(72) Inventor: Wahle, Stephan, 50670 Köln (DE); Schorr, Joachim, 40724 Hilden (DE); Weber, Martin, 42799 Leichlingen (DE)
(74) Representative: VOSSIUS & PARTNER

(56) References cited:
- WO-A-92/13963
- WO-A-95/21178
- CAPLEN N J ET AL: "GENE THERAPY FOR CYSTIC FIBROSIS IN HUMANS BY LIPOSOME-MEDIATED DNATRANSFER: THE PRODUCTION OF RESOURCES AND THE REGULATORY PROCESS" GENE THERAPY, vol. 1, no. 2, March 1994 (1994-03), pages 139-147, XP000676945
- HORN N A ET AL: "CANCER GENE THERAPY USING PLASMID DNA: PURIFICATION OF DNA FOR HUMAN CLINICAL TRIALS" HUMAN GENE THERAPY, vol. 6, no. 5, 1 May 1995 (1995-05-01), pages 565-573, XP000572705
- DATABASE WPI Section Ch, Week 8516 Derwent Publications Ltd., London, GB; Class B04, AN 85-095786 XP002093178 & JP 60 043383 A (TOYOBO KK), 7 March 1985 (1985-03-07)
- SAYERS, JR; EVANS, D; THOMSON, JB.: "Identification and Eradication of a Denatured DNA Isolated during Alkaline Lysis-Based Plasmid Purification Procedures" ANLYTICAL BIOCHEMISTRY, vol. 241, 1996, pages 186-189, XP002093176
- FELICIELLO, I.; CHINALI, G.: "A Modified Alkaline Lysis Method for the Preparation of Highly Purified Plasmid DNA from Escherichia coli" ANALYTICAL BIOCHEMISTRY, vol. 212, 1993, pages 394-401, XP002093177
- HOLMES D S ET AL: "A RAPID BOILING METHOD FOR THE PREPARATION OF BACTERIAL PLASMIDS" ANALYTICAL BIOCHEMISTRY, vol. 114, no. 1, June 1981 (1981-06), pages 193-197, XP000675212
- PRAZERES D M F ET AL: "Preparative purification of supercoiled plasmid DNA using anion -exchange chromatography" JOURNAL OF CHROMATOGRAPHY A, vol. 806, no. 1, 8 May 1998 (1998-05-08), page 31-45 XP004121166
- SAMBROOK J FRITSCH E F MANIATIS T: "PURIFICATION OF PLASMID DNA. PURIFICATION OF PLASMID DNA BY PRECIPITATION WITH POLYETHYLENE GLYCOL" 1989 , PAGE(S) 140/141, 146/147 XP002049730
- SOHAIL A ET AL: "AN IMPROVED METHOD FOR ISOLATION OF COVALENTLY CLOSED CIRCULAR DNA OX174AM3 PHAGE" PAKISTAN JOURNAL OF ZOOLOGY, vol. 19, no. 4, 1987, pages 407-412, XP000197613

## Description

The present invention relates to a method of producing covalently closed circular (ccc) DNA from a unicellular organism, preferably a bacterium, essentially free of genomic DNA. The method of the invention comprises the steps of precipitating a cleared lysate with isopropanol and, after washing and resuspension of the precipitate, the digestion of the genomic DNA with a nuclease that cleaves linear DNA or circular DNA that comprises a nick or comprises a free 3'- or 5'-end but not ccc DNA. Finally, the purified ccc DNA is separated from the remainder of the product of the digestion step by contacting said product with an ion exchange material. Preferably, the separation step is a chromatography using an anion exchange column. The invention also relates to purified ccc DNA obtained by the method of the invention, pharmaceutical compositions comprising said ccc DNA, as well as to a kit for carrying out the method of the invention.

The production of covalently closed circular DNA in pure form has become increasingly important in the past two decades. The development of DNA vaccines in the last few years has added to this demand. The purification protocol for ccc DNA requires its separation from the genomic DNA of the unicellular organism carrying the ccc DNA. Standard protocols designed to simplify this process as far as possible have the problem that after the degradation step for genomic DNA the sample still comprises the degrading protein or fragments thereof. In WO92/13963 a method for purification of ccc DNA is described which comprises a nuclease digestion step in order to remove contaminating DNA and a subsequent ion exchange purification step of the ccc DNA. Thus, Epicentre Technologies, Madison, Wisconsin, USA, distributes an ATP-dependent DNAse under the tradename Plasmid-Safe™ for the separation of plasmid DNA from genomic DNA. The enzyme specifically cleaves linear DNA (such as genomic DNA) while leaving ccc DNA intact. The earliest identification of an enzyme of this class, namely of the ATP-dependent exonuclease RecBCD was published in 1972 (Goldmark and Linn, J. Biol. Chem. 247, 1849-1860).

The proteinaceous impurities contained in the ccc DNA sample solutions generate problems when the ccc DNA is used for subcloning purposes. In addition, they are expected to cause adverse side effects when finding application in *in vivo* administrations.

Thus, the technical problem underlying the present invention was to improve the standard protocols for separating ccc DNA from genomic DNA. In accordance with the present invention it was found that a novel process comprising a sequel of steps discussed in more detail below not only provides ccc DNA of a high purity grade but also removes proteinaceous impurities that could interfere with subsequent cloning steps or *in vivo* applications.

Accordingly, the present invention relates to a method of producing covalently closed circular (ccc) DNA from a unicellular organism wherein said ccc DNA is essentially free of genomic DNA comprising the steps of:
(a) precipitation of a cleared lysate of said unicellular organism with 0.6 - 5 volumes of alcohol;
(b) washing of the precipitate with an alcoholic solution;
(c) resuspension of the precipitate;
(d) digestion of the genomic DNA with a nuclease that cleaves linear DNA or/and circular DNA that comprises a nick or comprises a free 3'- or 5'-end but not ccc DNA; and
(e) separating purified ccc DNA from the remainder of the product of step (d) by contacting the product of step (d) with an anion exchange material.

The term "covalently closed circular DNA" as used herein refers to DNA molecules that have assumed a circular form in contrast to linear DNA molecules such as eukaryotic chromosomal DNA or bacterial chromosomal DNA that comprises a nick or comprises a free 3'- or 5'-end. Moreover, the circular structure of the above referenced DNA molecules is covalently closed. ccc DNA is well known in the art and is further described, for example, in KG. Hardy (ed) "Plasmids, a practical approach", IRL Press Oxford U.K., Washington D.C., U.S.A., 1987. The term "essentially free of genomic DNA" is intended to mean that more than 95% of the genomic DNA, preferably more than 98%, and most preferably more than 99% of the genomic DNA in the sample has been degraded and removed. Optimally, the ccc DNA is close to 100% purified or to 100% purified.
The term "cleared lysate" is also well known in the art and bears the meaning of an aqueous solution containing plasmid DNA, RNA and proteins which is obtained after lysis of unicellular organims and the separation of the cell debris usually by filtration or centrifugation. For obtaining a cleared lysate of a unicellular organism, lysis of said organism has to precede the steps recited above. Accordingly, in one embodiment, the method of the invention envisages an additional step that relates to the lysis of said unicellular organism under conditions that leave the ccc DNA essentially intact. Methods for the lysis of said organisms are well know in the art and described, for example, in Sambrook et al. "Molecular Cloning, A Laboratory Handbook" 2^{nd} edition 1989, CSH Press, Cold Spring Harbor, U.S.A.

The invention has the particular advantage that highly purified ccc DNA is obtained using a non-sophisticated and rather in-expensive method. Yet, the sequel of steps is crucial and can by no means be regarded as obvious. For example, when starting out in finding a solution to the above referenced technical problems, the inventors tried in vain to establish the step of employing the DNase directly after the lysis of bacteria by alkali treatment. This negative result is probably due to the presence of impurities, or salts etc. in the lysate. The method of the current invention saves several hours of preparation time compared to the conventional procedures of adding the enzyme to a final stage of the proceedings. Especially advantageous is the fact that with the current invention the use of toxic reagents like phenol or chloroform for the extraction of the remaining enzyme can be totally avoided.

The ccc DNA obtained by the method of the invention can be used for a variety of purposes including cloning procedures and pharmaceutical applications such as vaccination. The ccc DNA may optionally be further purified, for example, by HPLC or may be prepared for storage, e.g., by lyophilisation.

As is well known in the art, there are different forms of ccc DNA. In accordance with the present invention, it is preferred that said ccc DNA is a plasmid. As is also well known, plasmids are often used as vectors. The method of the invention has turned out to be particular advantageous for producing highly purified plasmids and in particular for plasmids with low copy numbers.
Preferably, said vector is an expression vector and/or a gene transfer or targeting vector. Expression vectors derived from viruses such as retroviruses, vaccinia virus, adeno-associated virus, herpes viruses, or bovine papilloma virus, may be used for delivery of the vector into targeted cell populations. Methods which are well known to those skilled in the art can be used to construct recombinant viral vectors; see, for example, the techniques described in Sambrook et al. loc. cit. and Ausubel, Current Protocols in Molecular Biology, Green Publishing Associates and Wiley Interscience, N.Y. (1989). Alternatively, the vectors can be reconstituted into liposomes for delivery to target cells. The vectors can be transferred into the host cell by well-known methods, which vary depending on the type of cellular host. For example, calcium chloride transfection is commonly utilized for prokaryotic cells, whereas calcium phosphate treatment or electroporation may be used for other cellular hosts; see Sambrook, supra.
Such vectors may comprise further genes such as marker genes which allow for the selection of said vector in a suitable host cell and under suitable conditions. Preferably, the vector comprises polynucleotide sequences which are operatively linked to expression control sequences allowing expression in prokaryotic or eukaryotic cells. Expression of said polynucleotide comprises transcription of the polynucleotide into a translatable mRNA. Regulatory elements ensuring expression in eukaryotic cells, preferably mammalian cells, are well known to those skilled in the art. They usually comprise regulatory sequences ensuring initiation of transcription and optionally poly-A signals ensuring termination of transcription and stabilization of the transcript. Additional regulatory elements may include transcriptional as well as translational enhancers, and/or naturally-associated or heterologous promoter regions. Possible regulatory elements permitting expression in prokaryotic host cells comprise, e.g., the PL, lac, trp or tac promoter in E. coli, and examples for regulatory elements permitting expression in eukaryotic host cells are the AOX1 or GAL1 promoter in yeast or the CMV-, SV40- RSV-promoter (Rous sarcoma virus), CMV-enhancer, SV40-enhancer or a globin intron in mammalian and other animal cells. Beside elements which are responsible for the initiation of transcription such regulatory elements may also comprise transcription termination signals, such as the SV40-poly-A site or the tk-poly-A site, downstream of the polynucleotide. Furthermore, depending on the expression system used leader sequences capable of directing the polypeptide to a cellular compartment or secreting it into the medium may be added to the coding sequence of the polynucleotide of the invention and are well known in the art. The leader sequence(s) is (are) assembled in appropriate phase with translation, initiation and termination sequences, and preferably, a leader sequence capable of directing secretion of translated protein, or a portion thereof, into the periplasmic space or extracellular medium. Optionally, the heterologous sequence can encode a fusion protein including an C- or N-terminal identification peptide imparting desired characteristics, e.g., stabilization or simplified purification of expressed recombinant product. In this context, suitable expression vectors are known in the art such as Okayama-Berg cDNA expression vector pcDV1 (Pharmacia), pCDM8, pRc/CMV, pcDNA1, pcDNA3 (In-vitrogene), or pSPORT1 (GIBCO BRL).
Preferably, the expression control sequences will be eukaryotic promoter systems in vectors capable of transforming or transfecting eukaryotic host cells, but control sequences for prokaryotic hosts may also be used.
As mentioned above, the vector of the present invention may also be a gene transfer or targeting vector. Gene therapy, which is based on introducing therapeutic genes into cells by ex-vivo or in-vivo techniques is one of the most important applications of gene transfer. Suitable vectors and methods for in-vitro or in-vivo gene therapy are described in the literature and are known to the person skilled in the art; see, e.g., Giordano, Nature Medicine 2 (1996), 534-539; Schaper, Circ. Res. 79 (1996), 911-919; Anderson, Science 256 (1992), 808-813; Isner, Lancet 348 (1996), 370-374; Muhlhauser, Circ. Res. 77 (1995), 1077-1086; Wang, Nature Medicine 2 (1996), 714-716; WO94/29469; WO 97/00957 or Schaper, Current Opinion in Biotechnology 7 (1996), 635-640. The polynucleotides and vectors of the invention may be designed for direct introduction or for introduction via liposomes, or viral vectors (e.g. adenoviral, retroviral) into the cell. Preferably, said cell is a germ line cell, embryonic cell, or egg cell or derived therefrom, most preferably said cell is a stem cell.

In an alternative preferred embodiment, said ccc DNA is cosmid DNA, P1 (P1 is a construct derived from a P1 phage, described e.g. in Ausubel, loc. cit.) DNA, BAC (Bacterial Artificial Chromosome) DNA or PAC (Phage Artificial Chromosome) DNA. The technical details and applications discussed above for plasmids in many cases also apply to the above referenced DNAs which is well known to the person skilled in the art. The method of the invention has turned out to be also particularly advantageous for the production of highly purified cosmid DNA, P1, BAC DNA or PAC DNA. This is due to the fact that cosmid DNA or the other DNA types referred to here is, as compared to "normal" plasmid DNA, present in a cell only in low copy numbers. Therefore, the percentage of genomic DNA in the overall amount of DNA is rather high. This high ratio is especially disadvantageous for a variety of applications, for example, the introduction of cosmid, P1, BAC or PAC DNA into *in vitro* cultured cells. Further, subcloning experiments will be accompanied by a high background of clones carrying genomic bacterial DNA inserts. Furthermore, the preparation of plasmids/cosmids or the other referenced DNAs according to GLP/GMP standards is significantly simplified by incorporating the method of the invention into this preparation process.

Whereas the unicellular organism may be a eukaryote such as yeast or Aspergillus or an archaebacterium, it is preferred in accordance with the present invention that said unicellular organism is a bacterium. In a particularly preferred embodiment, said bacterium is E. coli.

As has been stated above, the precipitation of plasmid or cosmid or other DNA such as specified above as P1, BAC or PAC DNA, RNA and proteins from the cleared lysate can be effected within a certain range of volumes of alcohol added which is between 0.6 and 5 volumes, preferably 0.6 to 3 volumes. Examples of preferred alcohols are isopropanol and ethanol. Preferably, the quantum of isopropanol employed is about 0.6 times the volume of the lysate or a solution comprising said lysate and more preferably 0.6 times said volume. Ethanol is preferably added in an amount of about 2.5 volumes and more preferably in an amount of 2.5 volumes.

The subsequent washing step is preferably carried out with a solution of ethanol in water which is most preferably a 70% ethanol solution. The washing step can be repeated one or more times. Subsequently to the washing step(s) it may be advantageous to at least partially dry the precipitate. This can be done by a variety of methods well known in the art.

In accordance with the present invention, it is additionally preferred that said resuspension in step (c) is carried out in a reaction buffer suitable for a high activity of the nuclease. Examples of these reaction buffers can be taken from the appended examples. However, the buffer conditions for a high activity can be adjusted by the person skilled in the art on the basis of his technical knowledge and the teachings of this invention without further ado. In one most preferred embodiment, the nuclease is added to the reaction buffer directly before resuspension is started. In another most preferred embodiment, the nuclease is added after the precipitate has been resuspended. Other embodiments in this regard, that the person skilled in the art might envisage, e.g. addition during resuspension, are likewise covered by the invention. Of course, the above embodiments also comprise adding a concentrate of the buffers in combination with a diluent, for example, deionized water.

The nuclease employed in the method of the invention may be an endonuclease or an exonuclease. In a further preferred embodiment of the method of the invention, said nuclease that cleaves linear DNA, circular DNA that comprises a nick or comprises a free 3'- or 5'-end but not ccc DNA is an ATP dependent exonuclease. Most preferably, said ATP dependent exonuclease is the exonuclease RecBCD (EC 3.1.11.5).
A particular advantage of this type of enzyme is the dependency of its activity on ATP. Consequently, the investigator has a distinct means in his hands to trigger, direct or stop the degradation of the genomic DNA.

It is furthermore preferred in accordance with the present invention that said separation in step (e) is effected by chromatographing said product of step (d) over an anion exchange column and recovering said purified ccc DNA in the eluate.
This embodiment is particularly advantageous since large batches of product can be purified to a high degree of purity using a convenient procedure that may be automated. The column will retain impurities such as nuclease or fragments thereof as well as other proteinaceous material that may have remained in the sample from the precipitation step. The eluted material comprises the highly purified ccc plasmid DNA which may be further processed, for example, bottled, freeze-dried, precipitated etc., according to conventional protocols.

Those embodiments are particularly preferred, wherein said anion exchange material is based on a polymeric inorganic support material, such as acryl resin, dextran agarose or a combination thereof, wherein preferably the groups bound to the anion exchanger have a surface charge from 1 to 4 µM/ml of support surface area, corresponding to the 0.5 to 500 µM/ml. The chromatographic support material suggested in German patent application P 44 03 692 may be preferably used as a modified porous or non-porous inorganic and/or organic material. Further, anion exchange material, such as QIAGEN DEAE Sepharose, Q Sepharose, DEAE Sephadex, Phoros 20 M/P or Phoros 50 M/P or Hypher D may be used.

### Trademarks

Patented or patent-pending technology and/or registered or registration-pending trademark of QIAGEN: QIAGEN®.

Beckman is a registered trademark of Beckman Instruments, Inc.

Triton is a registered trademark of Rohm and Haas Inc.

The examples illustrate the invention.

### Example 1: Preparation of BAC/PAC/P1/Cosmid DNA

This protocol is for preparation of up to 50 µg of BAC, PAC, P1 or up to 200 µg of cosmid DNA using a QIAGEN-tip 500 (Maxi).

| **Materials:** | |
|---|---|
| **BAC/PAC/P1/Cosmid** | |
| **Maxi Kit (5)** | |
| QIAGEN-tip 500 | 5 |
| Buffer P1 | 110ml |
| Buffer P2 | 110ml |
| Buffer P3 | 110 ml |
| Buffer QBT | 60 ml |
| Buffer QC | 2x 205 ml |
| Buffer QF | 110 ml |
| RNase A | 11 mg (100 mg/ml) |
| ATP-dependent | 5x 115 µg |
| Exonuclease | |
| ATP solution (100 mM) | 1.75 ml |
| Reaction Buffer | 60 ml |
| Buffer QS (Adjustment Buffer) | 60 ml |
| Exonudease Buffer | 1.5 ml |
| Folded filters | 5 |
| Protocol | 1 |

| **Recommended culture volumes** | |
|---|---|
| | QIAGEN-tip 500 |
| BAC | 500 ml |
| PAC | 500 ml |
| P1 | 500 ml |
| Cosmid | 500 ml |

**The following steps were taken before starting the actual experiments:**
(a) Add RNase A solution to Buffer P1 before use. Use one vial of RNase A (spin down briefly before use) per bottle of Buffer P1, to give a final concentration of 100 µg/ml.
(b) Use one vial of ATP-dependent Exonuclease per preparation. Immediately before use, resuspend the Exonuclease of each vial in 225 µl of Exonuclease Buffer.
(c) Check Buffer P2 for SDS precipitation due to low storage temperatures. If necessary, dissolve the SDS by warming to 37°C.
(d) Pre-chill Buffer P3 to 4°C.

### Procedure

1. A single was picked colony from a freshly streaked selective plate and a starter culture of 2-5 ml LB-Miller medium containing the appropriate selective antibiotic inoculated. It was then incubated for ∼8 hours at 37°C with vigorous shaking (∼300 rpm).
   A flask with a volume of at least 4 times the volume of the culture was used.
2. The starter culture was diluted 1/500 to 1/1000 into 500 ml selective LB-Miller medium. It was then grown at 37°C for 12-16 hours with vigorous shaking (∼300 rpm).
   A flask or vessel with a volume of at least 4 times the volume of the culture was used. The culture should reach a cell density of approximately 1 x 10⁹ cells per ml.
3. The bacterial cells were harvested by centrifugation at 6000 x *g* for 15 min at 4°C.
   6000 x *g* corresponds to 6000 rpm in Sorvall GSA or GS3 or Beckman® JA-10 rotors. All traces of supernatant were removed by inverting the open centrifuge tube until all medium had been drained.
4. The bacterial pellet was resuspended in 20 ml of Buffer P1.
   Care was taken that RNase A is added to Buffer P1. The bacteria were resuspended completely leaving no cell clumps.
   Optionally, the resuspended bacteria can be split into 2x 10 ml. Then both samples are processed through steps 5 and 6, and the samples reunified after step 7, before the filtration.
5. 20 ml of Buffer P2 were added, mixed gently but thoroughly by inverting 4-6 times, and incubated at room temperature for 5 min.
   Vortexing is avoided, as this will result in shearing of genomic DNA. The lysate should appear viscous. The lysis reaction is not allowed to proceed for more than 5 min. After use, the bottle containing Buffer P2 was closed immediately to avoid acidification of Buffer P2 from CO₂ in the air.
6. 20 ml of chilled Buffer P3 was added, mixed immediately but gently by inverting 4-6 times, and incubated on ice for 10 min.
   Precipitation was enhanced by using chilled Buffer P3 and incubation on ice. After addition of Buffer P3, a cloudy and very viscous precipitate containing genomic DNA, proteins, cell debris, and SDS became visible. Thorough mixing avoids localized potassium dodecyl sulfate precipitation.
7. Centrifugation was carried out at ≥20,000 x *g* for 30 min at 4°C. The supernatant containing BAC/PAC/P1/Cosmid DNA was removed promptly.
   Before loading the centrifuge, the sample was mixed again. Centrifugation was performed in non-glass tubes (e.g., polypropylene). A centrifugal force of 20,000 x *g* corresponds to 12,000 rpm in a Beckman JA-17 rotor or 13,000 rpm in a Sorvall SS-34 rotor. After centrifugation, the supernatant was and should be clear.
   If two samples have been processed in parallel since step 4, they should be reunified now for step 8.
8. The lysate was filtered through a A. dest. prewetted folded filter.
   This filtration step is carried out to avoid precipitation of suspended or particulate material in step 9, which could lead to suboptimal Exonuclease performance in step 12.
9. The DNA was precipitated by adding 35 ml (0.6 volumes) room-temperature isopropanol to the cleared lysate. It was then mixed and centrifuged immediately at ≥15,000 x *g* for 30 min at 4°C. The supernatant was then carefully decanted.
   Isopropanol should be at room temperature in order to minimize salt precipitation, although centrifugation was carried out at 4°C to prevent overheating of the sample. A centrifugal force of 15,000 x *g* corresponds to 9,500 rpm in a Beckman JS-13 rotor and 11,000 rpm in a Sorvall SS-34 rotor. This isopropanol pellet contains proteins and genomic DNA and is therefore typically easy to see.
   Optionally, the cleared lysate can alternatively be split into 2x 50 ml tubes and centrifuged at 5000 x g for 60 minutes in an e.g. Heraeus Minifuge. Both samples should be continued to be processed through step 10 and the samples reunified in step 11, before Exonuclease digestion.
10. The DNA pellet was washed with 5 ml of room-temperature 70% ethanol, and centrifuged at >15,000 x *g* for 15 min. Carefully the supernatant was decanted without disturbing the pellet.
   The 70% ethanol removes precipitated salt and replaces isopropanol with the more volatile ethanol, making the DNA easier to redissolve.
11. The tube containing the DNA pellet was placed upside down on a paper towel and the DNA was allowed to air-dry for 2-3 min. Any additional liquid that may be visible on the tube opening was carefully removed and carefully the DNA redissolved in 9.5 ml of Reaction Buffer, until the DNA was completely dissolved.
   Dissolving the DNA should only take place by very gentle shaking.
   If two samples have been processed in parallel since step 9, they should be reunified now for step 12.
12. 200 µl of ATP-dependent Exonuclease (previously dissolved in Exonuclease Buffer) and 300 µl ATP Solution (100 mM ATP solution) was added to the dissolved DNA, mixed gently but thoroughly, and incubated in a water bath or heating block at 37°C for 60 minutes.
   During this step genomic DNA and nicked BAC/PAC/P1/Cosmid DNA will be digested by the exonuclease. Only supercoiled DNA will remain for further purification.
13. A QIAGEN-tip 500 was equilibrated by applying 10 ml Buffer QBT, and allowed the column to empty by gravity flow.
   Flow of buffer will begin automatically by reduction in surface tension due to the presence of detergent in the equilibration buffer. The QIAGEN-tip was allowed to drain completely. QIAGEN-tips can be left unattended, since the flow of buffer will stop when the meniscus reaches the upper frit in the column.
14. 10 ml of QS Buffer was added to the DNA sample from step 12, the whole sample applied to the QIAGEN-tip and allowed to enter the resin by gravity flow.
15. The QIAGEN-tip was washed with 2 x 30 ml Buffer QC.
16. The DNA was eluted with 15 ml Buffer QF, prewarmed to 65°C.
   Use of prewarmed buffer QF will enhance efficient elution of large DNA molecules. The eluate was collected in a 30 ml tube. Use of polycarbonate centrifuge tubes is not recommended as polycarbonate is not resistant to the alcohol used in subsequent steps.
17. DNA was precipitated by adding 10.5 ml (0.7 volumes) room-temperature isopropanol to the eluted DNA, then mixed and centrifuged immediately at ≥15,000 x g for 30 min at 4°C. The supernatant was carefully decanted.
   All solutions were and should be at room temperature in order to minimize salt precipitation, although centrifugation is carried out at 4°C to prevent overheating of the sample. A centrifugal force of 15,000 x *g* corresponds to 9,500 rpm in a Beckman JS-13 rotor and 11,000 rpm in a Sorvall SS-34 rotor. Isopropanol pellets have a glassy appearance and may be more difficult to see than the fluffy, salt-containing pellets that result from ethanol precipitation. Marking the outside of the tube before centrifugation allows the pellet to be more easily located. Isopropanol pellets are also more loosely attached to the side of the tube, and care should be taken when removing the supernatant.
18. The DNA pellet was washed with 5 ml of room-temperature 70% ethanol, and centrifuged at >15,000 x *g* for 15 min. The supernatant was carefully decanted without disturbing the pellet.
   The 70% ethanol removes precipitated salt and replaces isopropanol with the more volatile ethanol, making the DNA easier to redissolve.
19. The pellet was air-dried for 5-10 min, and the DNA redissolved in a suitable volume of buffer (e.g., TE, pH 8.0, or 10 mM Tris, pH 8.5).
   The DNA pellet was redissolved by rinsing the walls to recover all the DNA, especially if glass tubes have been used. Pipetting the DNA up and down to promote resuspension may cause shearing and should be avoided. Overdrying the pellet will make the DNA difficult to redissolve. The DNA may also be difficult to dissolve if it is too acidic. DNA dissolves best under slightly alkaline conditions.

| **Composition of buffers** | | |
|---|---|---|
| **Buffer** | **Composition** | **Storage** |
| Buffer P1 (Resuspension Buffer) | 50 mM Tris-HCl, pH 8.0; 10 mM EDTA; 100 µg/ml RNase A | 4°C, after addition on RNase A |
| Buffer P2 (Lysis Buffer) | 200 mM NaOH, 1% SDS | room temp. |
| Buffer P3 (Neutralization Buffer) | 3.0 M potassium acetate | room temp. or 4°C |
| Buffer QBT (Equilibration Buffer) | 750 mM NaCl; 50 mM MOPS, pH 7.0; 15% isopropanol; 0.15% Triton® X-100 | room temp. |
| Buffer QC (Wash Buffer) | 1.0 M NaCl; 50 mM MOPS, pH 7.0; 15% isopropanol | room temp. |
| Buffer QF (Elution Buffer) | 1.25 M NaCl; 50 mM Tris, pH 8.5; 15% isopropanol | room temp. |
| Buffer QS (Adjustment Buffer) | 1.5 M NaCl; 100 mM MOPS, pH 7.0; 15% isopropanol | room temp. |
| Exonuclease Buffer | 20 mM KCl; 20 mM potassium-phosphate, pH 7.5 | room temp. |
| Reaction Buffer | 50 mM Tris-HCl; 10 mM MgCl ₂ pH 8.5 | room temp. |
| TE | 10 mM NaCl; 10 mM Tris-HCl, pH 8.0; 1 mM EDTA | room temp. |

### Example 2: Preparation of about 100 mg Genomic free Plasmid DNA

66g biomass of E. coli bacteria containing the plasmid pCMVS2.S (Whalen et al., Proc. Natl. Acad. Sci. USA 92 (1995), 5307-5311 was lysed with 1L buffers of each P1, P2 and P3 according to EP 95 90 8258.7 corresponding to EP-A 0 743 949.
After lysis the cell debris was removed as described in EP 95 90 8259.5 corresponding to EP-A 0 781 291 or by centrifugation at 20.000 x g.
The plasmid DNA was precipitated by adding 0.6 vol. (2.25 l) of isopropanol and centrifuged at 20.000 x g for 30 min. The pellet was redissolved in 100 ml of reaction buffer (see Example 1), 30 ml ATP (100 mM ATP solution) solution and 10 mg of RecBCD enzyme redissolved in 20 ml of exonuclease buffer (20 mM KCI, 20 mM potassium phosphate, pH 7.5) was added to the solution and incubated for 120 mm. at 37°C. The solution was diluted with 150 ml of buffer QS and loaded onto a QIAGEN Ultrapure 100 anion exchange column, QIAGEN GmbH, Hilden, which had been equilibrated with 750 mM NaCI and processed following the Ultrapure 100 protocol provided by the manufacturer.

The above procedure results in a DNA which has less than 1% genomic DNA and consists of 99% supercoil molecules. The anion exchange purification removes the RecBCD enzyme with a 99% efficacy. This DNA fulfills the QC standards to be used in clinical applications for Gene Therapy and DNA Vaccination.

The actual amount of ccc DNA obtained may vary, for example depending on the plasmid isolated.

## Claims

1. A method of producing covalently closed circular (ccc) DNA from a unicellular organism wherein said ccc DNA is essentially free of genomic DNA comprising the steps of:
(a) precipitation of a cleared lysate of said unicellular organism with 0.6 to 5 volumes of alcohol;
(b) washing of the precipitate with an alcoholic solution;
(c) resuspension of the precipitate;
(d) digestion of the genomic DNA with exonuclease RecBCD (EC 3.1.11.5); and
(e) separating purified ccc DNA from the remainder of the product of step (d) by contacting the product of step (d) with an anion exchange material.

2. The method of claim 1, wherein said ccc DNA is plasmid DNA.

3. The method of claim 1, wherein said ccc DNA is cosmid DNA, P1 DNA, BAC DNA or PAC DNA.

4. The method of any one of claims 1 to 3, wherein said unicellular organism is a bacterium.

5. The method of claim 4, wherein said bacterium is E. coli.

6. The method of any one of claims 1 to 5, wherein precipitation in step (a) is effected with isopropanol or ethanol.

7. The method of any one of claims 1 to 6, wherein said alcoholic solution in step (b) is an ethanol solution in water.

8. The method of claim 7, wherein said ethanol solution is a 70% ethanol solution.

9. The method of any one of claims 1 to 8, wherein said resuspension in step (c) is carried out in a reaction buffer suitable for a high activity of said nuclease.

10. The method of claim 9, wherein said reaction buffer comprises the nuclease.

11. The method claim 9, wherein the nuclease is added to said resuspension product of step (c).

12. The method of any one of claims 1 to 11, wherein said separation in step (e) is effected by chromatographing said product of step (d) over an anion exchange column and recovering said purified ccc DNA in the eluate.

13. The method of any one of claims 1 to 12, wherein said anion exchange material is based on a polymeric inorganic support material, preferably an acryl resin, dextran agarose or a combination thereof, wherein the groups bound to the anion exchanger have a surface charge from 1 to 4 µM/ml of support surface area, corresponding to the 0.5 to 500 µM/ml.

## Revendications

1. Procédé pour produire de l'ADN circulaire fermé de manière covalente (ccc) à partir d'un organisme unicellulaire, ledit ADN ccc étant essentiellement exempt d'ADN génomique, comprenant les étapes de:
(a) précipiter un lysat clarifié dudit organisme unicellulaire avec 0,6 à 5 volumes d'alcool,
(b) laver le précipité avec une solution alcoolique,
(c) remettre le précipité en suspension,
(d) digérer l'ADN génomique avec l'exonucléase RecBCD (EC 3.1.11.5), et
(e) séparer l'ADN ccc clarifié du reste du produit de l'étape (d) en mettant en contact le produit de l'étape (d) avec un matériau échangeur d'anions.

2. Procédé selon la revendication 1, dans lequel l'ADN ccc est de l'ADN plasmidique.

3. Procédé selon la revendication 1, dans lequel l'ADN ccc est de l'ADN cosmidique, ADN P1, ADN BAC ou ADN PAC.

4. Procédé selon l'une quelconque des revendications 1 à 3, dans lequel ledit organisme unicellulaire est une bactérie.

5. Procédé selon la revendication 4, dans lequel ladite bactérie est *E. coli.*

6. Procédé selon l'une quelconque des revendications 1 à 5, dans lequel la précipitation de l'étape (a) est réalisée avec de l'isopropanol ou de l'éthanol.

7. Procédé selon l'une quelconque des revendications 1 à 6, dans lequel ladite solution alcoolique de l'étape (b) est une solution d'éthanol dans de l'eau.

8. Procédé selon la revendication 7, dans lequel ladite solution d'éthanol est une solution d'éthanol à 70 %.

9. Procédé selon l'une quelconque des revendications 1 à 8, dans lequel ladite remise en suspension de l'étape (c) est réalisée dans un tampon de réaction adapté à une activité élevée de ladite nucléase.

10. Procédé selon la revendication 9, dans lequel ledit tampon de réaction comprend la nucléase.

11. Procédé selon la revendication 9, dans lequel ladite nucléase est ajoutée audit produit de remise en suspension de l'étape (c).

12. Procédé selon l'une quelconque des revendications 1 à 11, dans lequel ladite séparation de l'étape (e) est réalisée par chromatographie dudit produit de l'étape (d) sur une colonne échangeuse d'anions et par récupération dudit ADN ccc purifié dans l'éluat.

13. Procédé selon l'une quelconque des revendications 1 à 12, dans lequel ledit matériau échangeur d'anions est basé sur un matériau support inorganique polymérique, de préférence une résine acryle, un agarose de dextrane ou un mélange de ces produits, les groupes liés à l'échangeur d'anions ayant une charge de surface de 1 à 4 µM/ml d'aire de surface support, correspondant au 0,5 à 500 µM/ml.

## Patentansprüche

1. Verfahren zur Herstellung kovalent geschlossener ringförmiger (ccc) DNA von einem einzelligen Organismus, wobei die ccc-DNA im wesentlichen frei von genomischer DNA ist, umfassend die Schritte:
(a) Präzipitation eines gereinigten Lysats des einzelligen Organismus mit 0.6 bis 5 Volumina Alkohol;
(b) Waschen des Präzipitates mit einer alkoholischen Lösung;
(c) Resuspension des Präzipitates;
(d) Verdau der genomischen DNA mit Exonuclease RecBCD (EC 3.1.11.5); und
(e) Abtrennen der gereinigten ccc-DNA von dem übrigen Produkt aus Schritt (d) durch Inkontaktbringen des Produktes aus Schritt (d) mit einem Anionen-Austauschmaterial.

2. Verfahren nach Anspruch 1, wobei die ccc-DNA Plasmid-DNA ist.

3. Verfahren nach Anspruch 1, wobei die ccc-DNA Cosmid-DNA, P1-DNA, BAC-DNA oder PAC-DNA ist.

4. Verfahren nach einem der Ansprüche 1 bis 3, wobei der einzellige Organismus ein Bakterium ist.

5. Verfahren nach Anspruch 4, wobei das Bakterium E. coli ist.

6. Verfahren nach einem der Ansprüche 1 bis 5, wobei die Präzipitation in Schritt (a) mit Isopropanol oder Ethanol erfolgt.

7. Verfahren nach einem der Ansprüche 1 bis 6, wobei die alkoholische Lösung in Schritt (b) eine Ethanol-Lösung in Wasser ist.

8. Verfahren nach Anspruch 7, wobei die Ethanol-Lösung eine 70%ige Ethanol-Lösung ist.

9. Verfahren nach einem der Ansprüche 1 bis 8, wobei die Resuspension in Schritt (c) in einem Reaktionspuffer ausgeführt wird, der geeignet ist für eine hohe Aktivität der Nuclease.

10. Verfahren nach Anspruch 9, wobei der Reaktionspuffer die Nuclease umfasst.

11. Verfahren nach Anspruch 9, wobei die Nuclease zu dem Resuspensionsprodukt aus Schritt (c) zugesetzt wird.

12. Verfahren nach einem der Ansprüche 1 bis 11, wobei die Abtrennung in Schritt (e) durch Chromatographieren des Produktes aus Schritt (d) über eine Anionen-Austauschsäule und Wiedergewinnen der gereinigten ccc-DNA in dem Eluat ausgeführt wird.

13. Verfahren nach einem der Ansprüche 1 bis 12, wobei das Anionen-Austauschmaterial auf einem polymeren anorganischen Trägermaterial basiert, vorzugsweise ein Acrylharz, Dextranagarose oder eine Kombination davon, wobei die Gruppen, die an den Anionen-Austauscher gebunden sind, eine Oberflächenbeladung von 1 bis 4 µM/ml des Trägeroberflächenbereichs haben, entsprechend 0,5 bis 500 µM/ml.
